# EUROPEAN PATENT SPECIFICATION

(11) **EP 4 460 338 B1**
(45) Date of publication and mention of the grant of the patent: **16.07.2025**
(21) Application number: 22839805.3
(22) Date of filing: 19.12.2022
(51) Int. Cl.: A61L 2/00, A61L 2/08, A61L 2/10, A61L 2/20, A61L 9/20

(54) **A RADIATION GENERATING SYSTEM COMPRISING AN OPTICAL ARRANGEMENT FOR A FAR UV LIGHT SOURCE MINIMIZING THE IMPACT OF UNFILTERED UNDESIRED WAVELENGTHS**
STRAHLUNGSERZEUGUNGSSYSTEM MIT EINER OPTISCHEN ANORDNUNG FÜR EINE FERN-UV-LICHTQUELLE ZUR MINIMIERUNG DER AUSWIRKUNGEN UNGEFILTERTER UNERWÜNSCHTER WELLENLÄNGEN
SYSTÈME DE GÉNÉRATION DE RAYONNEMENT COMPRENANT UN AGENCEMENT OPTIQUE POUR UNE SOURCE DE LUMIÈRE ULTRAVIOLETTE LOINTAINE MINIMISANT L'IMPACT DES LONGUEURS D'ONDE INDÉSIRABLES NON FILTRÉES

(30) Priority: 04.01.2022 EP 22150110
(43) Date of publication of application: 13.11.2024
(73) Proprietor: Signify Holding B.V., 5656 AE Eindhoven (NL)
(72) Inventor: VAN BOMMEL, Ties, 5656 AE Eindhoven (NL); PET, Robert, Jacob, 5656 AE Eindhoven (NL)
(74) Representative: Verweij, Petronella Daniëlle
(86) International application number: PCT/EP2022/086757
(87) International publication number: WO 2023/131509

(56) References cited:
- EP-A2- 0 240 005
- US-A1- 2006 261 291
- US-A1- 2017 289 524
- US-A1- 2020 215 214

## Description

### TECHNICAL FIELD

The present invention relates to a radiation generating system. Further, the invention relates to a method for treating at least part of a space or of an object using such a radiation generating system.

### BACKGROUND

In view of the recent development in the world concerning the global pandemic, disinfection has become a topic of renewed interest as the demand for sterilization increases. One way of disinfecting involves the use of UV light. As a response to pathogenic outbreaks involving airborne microorganisms it would be beneficial to employ UV light for disinfecting air and objects at locations where the transmission of such microorganisms is believed to occur.

Normally, disinfection by UV light sources is used under controlled conditions in areas where humans or animals are not present during ongoing disinfection, such as at surgery theaters or the like. However, the increased demand for germicidal activities may involve operating UV light sources in environments with human presence, thus introducing a risk for unintentional irradiation by UV light. Therefore, disinfecting light sources, in particular those involving UV light, should possess reliable safety features in order to avoid potential exposure of humans or animals to the harmful irradiation.

Recent studies show that far-UVC light, i.e. irradiation having a wavelength in the range from 190 nm to 230 nm is very effective in inactivation of bacteria and/or viruses, and is also relatively safe, at least compared to near-UVC light having a wavelength in the range from 230 to 285 nm.

However, far-UVC light sources such as discharge lamps, e.g. Excimer lamps, also provide unsafe UVC wavelengths, i.e. light outside the 190-230 nm wavelength range. To improve safety of such light sources, a filter, in particular an interference filter, may be used. Nevertheless, due to the angle dependence of the filter, unsafe UVC wavelengths may still exit the filter under higher angles.

In view of the above, it is desirable to produce systems that provide radiation having specifically selected wavelengths only. It may be possible, for instance, to use a light source, a collimator, and an interference filter. However, the selectivity of the interference filter may depend on the angle of incidence. Although a collimator is used upstream of the interference filter, the rays may not be fully parallel. This may lead to a variety in angles of incidence, which may result in transmission of undesired wavelengths through the interference filter. In case of UV light, this may lead to people being unintentionally exposed to less desirable radiation.

Hence, it is an aspect of the invention to provide a radiation generating system that provide improved safety, wherein unfiltered irradiation having undesired wavelengths is eliminated or at least minimized.

US 2017/289524 disclose an optical system for collecting distance information within a field is provided. The optical system may include lenses for collecting photons from a field and may include lenses for distributing photons to a field. The optical system may include lenses that collimate photons passed by an aperture, optical filters that reject normally incident light outside of the operating wavelength, and pixels that detect incident photons. The optical system may further include illumination sources that output photons at an operating wavelength.

### SUMMARY

The present invention thus provides such a radiation generating system. The radiation generating system of the present invention may be particularly suitable for disinfecting spaces with high level of activity, such as a waiting room in a hospital or a veterinary clinic, a public space such as a library, an office, a department store or the like, as well as public transportation means, such as busses or trains.

In accordance with the present invention, a radiation generating system comprises a radiation unit comprising a light source, a first collimator, and an optical arrangement. The light source is configured to generate light source radiation having a first spectral power distribution having an intensity I_{1,1}, which may be referred to as a primary intensity, at (at least) a first wavelength λ₁ and an intensity I_{1,2}, referred to as a secondary intensity, at (at least) a second wavelength λ₂. The light source radiation has a source spatial light distribution, which may be an omni directional light distribution, or another light distribution. The term "spatial light distribution" relates to the way light is distributed from a light source.

The light source may comprise any source of radiation that emits light in the UV wavelength range. The system of the invention is especially relevant for light sources emitting non-parallel irradiation and having a range of wavelengths. In particular, the light source may comprise one or more of an excimer (or exciplex) discharge based light source. In embodiments, the light source may comprise a KrBr excimer lamp which may provide radiation at 207 nm ± 15 nm. In alternative embodiments, the light source may comprise a KrCl excimer lamp which may provide radiation at 222 nm ± 15 nm. The wavelength ranges indicated above may be based on the full-width at half maximum (FWHM) of the emission bands.

The light source may comprise solid state light source. Preferably, at least 80% of the light source radiation is within the first wavelength λ₁.

The term "light source" may also refer to a plurality of the same or a plurality of different light sources. Hence, e.g. the term "excimer discharge based light source" may also refer to a plurality of excimer discharge based light sources.

As mentioned above, the light source may comprise a solid state light source, such as light-emitting diode, LED, and/or a laser diode. The solid state light sources may be narrow band emitters, e.g. light sources having a FWHM <20 nm. Further, the light source may be a low pressure mercury plasma lamp or an excimer light source. The light source may comprise a plurality of LEDs. By the term "plurality" in the context of the present invention is meant two or more.

The term "LED" as used in the context of the present invention implies any type of LED known in the art, such as inorganic LED(s), organic LED(s), polymer/polymeric LEDs, violet LEDs, blue LEDs, optically pumped phosphor coated LEDs, optically pumped nano-crystal LEDs. As used herein, the term "LED" can encompass a bare LED die arranged in a cup, which may be referred to as a LED package. When UV-C light is used, the LED may be mounted in a cavity covered in a non-contact manner by an emission window made from quartz/fused silica.

The plurality of LEDs may comprise at least 10 LEDs, preferably at least 20 LEDs, more preferably at least 30 LEDs.

The first collimator is configured in a light receiving relationship with the light source, wherein the first collimator is configured to collimate the light source radiation into collimated light source radiation having an initial spatial light distribution (ISLD). The ISLD may be the same for the first wavelength λ₁ and the second wavelength λ₂.

The first collimator may be configured downstream or upstream of the light source. The first collimator may comprise a reflective collimator, especially a light reflective collimator. The first collimator may especially narrow the beam of radiation. The beam width may be expressed in the full width of the beam at half its maximum intensity (FWHM). The light source may emit light source radiation in substantially all directions. The first collimator essentially redirects part of the light source radiation into collimated light source radiation directed to the optical arrangement. Some light source radiation may already be directed to the optical arrangement and may not interact with the first collimator. Hence, the radiation that enters the optical arrangement may comprise both light source radiation and collimated light source radiation. Especially, collimated light source radiation may refer to either light source radiation directed to the optical arrangement or collimated light source radiation directed to the optical arrangement or to a combination of both. Hence, the collimated light source radiation is collimated relative to the light source radiation. The term "collimated" in this context indicates that the beam is more collimated than upstream of the first collimator, it does not necessarily indicate that all rays are substantially parallel. The first collimator may be a light mixing chamber or a reflector.

Further, the optical arrangement of the radiation generating system according to the present invention is configured downstream of the first collimator. Especially, the optical arrangement is configured downstream of the first collimator and the light source. As the first collimator in embodiments may comprise a reflective collimator, the light source may be positioned in between the first collimator and the optical arrangement. The optical arrangement may receive the collimated light source radiation from the first collimator, and optionally light source radiation from the light source. The optical arrangement comprises an optical filter and a diffuser.

The optical filter has a higher transmission for the first wavelength λ₁ than for the second wavelength λ₂, especially when irradiated under the predefined angle or range of angles. The optical filter may be selected for absorbing undesired wavelengths and transmitting desired wavelengths. Hence, in specific embodiments the optical filter may have a high(er) transmission for safe UV radiation.

In particular, at the predefined angle, the transmission for the first wavelength λ₁ may at least be two times higher than for the second wavelength λ₂. Alternatively, or additionally, the transmission for the first wavelength λ₁ may be at least 20% and the transmission for the second wavelength λ₂ may be at maximum 10%. In alternative embodiments, the transmission for the first wavelength λ₁ may be at least 50% and the transmission for the second wavelength λ₂ may be at maximum 20%, such as at maximum 10%.

Especially, the first wavelength λ₁ may be selected from the range of 190-230 nm, preferably 200-230 nm, more preferably 200-222 nm and the second wavelength λ₂ may selected from one or more of the range of 100-190 nm or the range of 230-300 nm, preferably 230-280 nm, more preferably 222-280 nm. In other words, the radiation within the first wavelength λ₁ is relatively safe UVC light. This type of UV light can be used in killing/inactivating viruses and/or bacteria. The radiation within the second wavelength λ₂ is UVC light that is very effective in killing/inactivating viruses and/or bacteria, but that is considered harmful for humans and animals. The terms "inactivating" and "killing" with respect to a virus may herein especially refer to damaging the virus in such a way that the virus can no longer infect and/or reproduce in a host cell, i.e., the virus may be (essentially) harmless after inactivation or killing.

The intensity of the radiation may be defined as radiant flux, in Watt, for instance measured in an integrating sphere, or with a goniometer. Germicidal effectiveness and photobiological safety limits may depend on irradiance levels. In embodiments, the intensity of the radiation may be defined as irradiance, in Watt per square meter. In embodiments, the irradiance may be measured at a predefined distance and a predefined angle to the optical axis, especially in embodiments 1 meter from the light source at an angle of 20° to the optical axis. However, in embodiments other intensity parameters may also be used, as herein especially relative intensities are applied. In specific embodiments, in an operational mode, the irradiance may be in the range of 1 µW/cm2 - 10 mW/cm2, for instance especially at 1 meter from the light source (even more especially within an angle of 20° to the optical axis). Hence, in embodiments the system may also be indicated as "disinfection system".

In particular, the dominant peak wavelength of the first wavelength λ₁ may 207 nm and/or 222 nm. Further, the dominant peak wavelength of the second wavelength λ₂ may be in a wavelength range from 285 nm to 295 nm and/or from 255 to 260 nm. By the term "dominant peak wavelength" is meant the maximum value of the wavelength of emitted light in the specified wavelength range, which is the maximum value of the wavelength range generated by the device.

In specific embodiments, the optical filter may comprise an interference filter. Interference filters are well known in the art. The interference filter may specifically transmit wavelengths in the range of 190-230 nm at the predefined angle. The interference filter may specifically absorb wavelengths in the range of 100-190 nm at the predefined angle.

Additionally, or alternatively, the interference filter may specifically reflect wavelengths in the range of 100-190 nm at the predefined angle. Especially, the interference filter may have a lower transmission for a wavelength in the range of 100-190 nm. Additionally, or alternatively, the interference filter may specifically absorb wavelengths in the range of 230-280 nm at the predefined angle or have a lower transmission for a wavelength in such range.

The specificity of interference filters depends on the angle of incidence. Radiation at an angle of incidence that differs from the predefined angle, may be subjected to different transmission and absorption behavior of the interference filter. The term "predefined angle" may especially refer to a range of angles at which the optical filter absorbs the specified wavelengths.

The optical filter is configured to filter at least part of the collimated light source radiation into filtered radiation comprising at least portion of the light source radiation of the first wavelength λ₁ having a first spatial light distribution (SLD1) and at least portion of the light source radiation of the second wavelength λ₂ having a second spatial light distribution (SLD2) different from the first spatial light distribution (SLD1). SLD2 may have a batwing spatial light distribution (measured in cross section).

The optical filter may have a transmittance with a high transmission for the first wavelength λ₁ in a predefined angle θ with θ being in a range of -30 to +30° with respect to the normal to the filter, and wherein the optical filter has substantially no transmittance for the second wavelength λ₂ in the predefined angle θ with θ being in the range of -30 to +30° with respect to the normal to the filter. On other words, far-UVC light directed substantially perpendicularly towards the optical filter will pass the optical filter, while the UVC light directed substantially perpendicularly towards the optical filter will either be absorbed or reflected by the optical filter. Alternatively, the predefined angle range may be from -20 to +20° or -40 to +40°.

The diffuser is configured downstream of the optical filter. The diffuser is configured to diffuse at least a part of the filtered radiation into diffused radiation comprising at least a portion of the light source radiation of the first wavelength λ₁ having a third spatial light distribution (SLD3) and at least portion of the light source radiation of the second wavelength λ₂ having a fourth spatial light distribution (SLD4) different from the second spatial light distribution (SLD2). In particular, the diffuser may be configured to diffuse at least 80% of the filtered radiation into diffused radiation. In other words, the diffuser redirects at least portion of the light source radiation of the second wavelength λ₂, i.e. the harmful radiation, to another direction, such that this portion of radiation may be collimated once more by the first collimator, and subsequently pass through the optical filter, wherein this radiation is filtered. The diffused radiation may have an asymmetrical beam shape.

The reflectivity R of the diffuser may be in the range from 15% to 35% for the light source radiation, preferably R is in the range from 16% to 33%, more preferably R is in the range from 17% to 31%, most preferably R is in the range from 18% to 30%. According to such an embodiment, the radiation generating system provides improved disinfection performance, safety and/or efficiency, since the inventors have found that a diffuser having the above-mentioned reflectivity range diffuses the harmful radiation well, while too much backscattering (i.e. reflection) of the diffuser is avoided, which otherwise may result in light loss thus impairing efficiency.

The diffuser may be made of an inorganic material such as for example a plate of roughened sapphire.

The optical arrangement may comprise a second collimator being arranged downstream of the diffuser. The second collimator may be a reflective collimator configured to collimate the diffused radiation into arrangement radiation. The arrangement radiation has an arrangement spatial light distribution. The arrangement radiation may be less or more collimated than the collimated light source radiation, depending on the starting point. Thus, if the starting point is a light mixing chamber, the arrangement radiation will be more collimated than the collimated light source radiation. On the other hand, if the starting point is a reflector, the arrangement radiation may be less collimated than the collimated light source radiation. Further, the arrangement radiation may have a narrower spatial light distribution than the diffused radiation. The arrangement radiation may thus be more collimated relative to the collimated light source radiation. Collimated radiation may refer to radiation having a narrower spatial light distribution downstream of the collimator compared to upstream of the collimator. The collimated radiation may have a larger spatial light distribution than parallel radiation.

The initial spatial light distribution (ISLD) of the collimated light source radiation may have a first FWHM1 (full-width-half-max), the arrangement spatial light distribution of the arrangement radiation may have a second FWHM2 (full-width-half-max), wherein FWHM2-FWHM1≥5 degrees, preferably FWHM2-FWHM1≥10 degrees, more preferably FWHM2-FWHM1≥15 degrees, most preferably FWHM2-FWHM1≥20 degrees.

An optical axis O1 of the collimated light source radiation may be aligned with a normal N to the optical filter. The optical axis O1 of the collimated light source radiation may be defined as an imaginary axis that runs through a center of the light source and through the second collimator. The normal N to the optical filter may be defined as an axis perpendicular to a surface of the optical filter configured for the incidence of the collimated radiation. Hence, in specific embodiments, the radiation generating system may be configured such that the optical axis O1 of the collimated light source radiation and the normal (N) to the optical filter have a first angle α1 equal to 0°. Further, α1 may be equal to or lower than 30°, preferably α1≤20°, more preferably α1≤10°. The optical axis may be defined as an imaginary line that defines the path along which light propagates through the system starting from the light generating element, here especially the light source.

**In** particular, the diffuser may be in optical contact with the optical filter. By using really flat surfaces, scrupulously clean, and by sliding them together so as to exclude dust, two surfaces may be made to contact, that is to say, they will come into contact so close that the reflection at the interface practically vanishes, and when once this has been achieved the surfaces adhere with very considerable force. Such surfaces are the to be in optical contact. **In** such an embodiment, the light loss due to reflection losses inherent to difference in refractive index with air are eliminated. Alternatively, or additionally, adhesive may be applied between the diffuser and the optical filter.

The radiation generating system offers the advantage of improved safety, wherein the risk of emitting wavelength being harmful to humans or animals is minimized or eliminated. To this end, light within the undesired and/or harmful wavelengths that may pass the optical filter at higher angles is homogenized and/or diffused to a wider angle range by the diffuser, thus providing diffused light. The diffused light is subsequently collimated by the first and/or the second collimator such that a safe beam of arrangement light is emitted.

Especially, a light reflective material has a light reflectivity in the range of 50-100 %, especially in the range of 70-100%, for light having a wavelength selected from the visible wavelength range. As can be derived from the above, this may apply for a wavelength range of at least 3 nm, especially a wavelength range of at least 5 nm, such as a wavelength range of at least 10 nm, like a wavelength range of at least 20 nm (within the range of 100-380 nm).

Note that a material may be reflective for one or more first wavelengths and absorb one or more second wavelengths, which may e.g. be the case with colored material. The term "light reflective material" may herein especially refer to materials which have a relatively high reflection, such as at least 70%, over a relatively high wavelength range, such as at least 3 nm, especially a wavelength range of at least 5 nm, such as a wavelength range of at least 10 nm, like a wavelength range of at least 20 nm, e.g. metal reflectors.

The terms "upstream" and "downstream" relate to an arrangement of items or features relative to the propagation of the light from a light generating means, such aa a light source, wherein relative to a first position within a beam of light from the light generating means, a second position in the beam of light closer to the light generating means is "upstream", and a third position within the beam of light farther away from the light generating means is "downstream".

The phrases "a wavelength", "the wavelength", "one or more wavelengths", "first wavelength λ₁" and "second wavelength λ₂" may especially indicate one wavelength or multiple wavelengths or a sub-wavelength range. In particular, the above-mentioned terms may refer to a plurality of wavelengths, such as a wavelength range of at least 3 nm, at least 5 nm, at least 10 nm, at least 20 nm. Hence, the term "first wavelength λ₁" may also refer to a plurality of wavelengths selected from the range of 190-230 nm and the term "second wavelength λ₂" may also refer to a plurality of wavelengths selected from one or more of the range of 100-190 nm or the range of 230-300 nm. Hence, the phrase "the first wavelength λ₁ selected from the range of 190-230 nm and the second wavelength λ₂ selected from the range of 100-190 nm or 230-280 nm", and similar phrases, may also indicate "one or more first wavelengths λ₁ selected from the range of 190-230 nm and one or more second wavelengths λ₂ selected from the ranges of 100-190 nm and/or 230-280 nm."

In specific embodiments, the term "first wavelength λ₁" may refer to a wavelength range, such as a range of at least 5 nm, such as a range of at least 10 nm, with in embodiments the first wavelength λ₁ in the middle of this range. In such embodiments, the afore-mentioned transmission applies to the full wavelength range in relation to the first wavelength(s).

In specific embodiments, the term "second wavelength λ₂" may refer to a wavelength range, such as a range of at least 5 nm, such as a range of at least 10 nm, even 10 more especially at least 20 nm, with in embodiments the second wavelength λ₂ in the middle of this range. In such embodiments, the afore-mentioned transmission applies to the full wavelength range in relation to the second wavelength(s).

In specific embodiments, the term "second wavelength λ₂" may refer to two wavelengths, one selected from the range of 100-190 nm and one selected from the 15 wavelength range of 230-280 nm.

In specific embodiments, the term "second wavelength λ₂" may refer to two wavelength ranges, one selected from the range of 100-190 nm and one selected from the wavelength range of 230-280 nm, and each wavelength range of the second wavelength λ₂ may individually selected from e.g. a range of at least 5 nm, such as a range of at least 10 nm, 20 even more especially at least 20 nm, with in embodiments the second wavelength λ₂ in the middle of this range. In such embodiments, the afore-mentioned transmission applies to the full wavelength ranges in relation to the second wavelength(s).

The ratio between an intensity I_{1,1} at a first wavelength λ₁ and an intensity I_{1,2} at a second wavelength λ₂ of a first spectral power distribution of the light source radiation may be between 0.1 and 100, i.e. 0.1≤I_{1,1}/I_{1,2}≤100.

In particular, I_{1,1}/I_{1,2}≥0.1, preferably I_{1,1}/I_{1,2}≥1, more preferably I_{1,1}/I_{1,2}≥2, most preferably I_{1,1}/I_{1,2}≥3. In this way, the radiation generating system may be more efficient compared to light sources having a lower relative intensity I_{1,1} at a first wavelength λ₁. Additionally, or alternatively, I_{1,1}/I_{1,2≤}100, preferably I_{1,1}/I_{1,2}≤50, more preferably I_{1,1}/I_{1,2}≤20, most preferably I_{1,1}/I_{1,2}≤15. In this way, the radiation system may be more relevant as there may actually be radiation to filter. Hence, in specific embodiments, I≤ I_{1,1}/I_{1,2}≤50, especially 2< I_{1,1}/I_{1,2}≤20.

The radiation generating system of the present invention may comprise a beam-shaping optical element being arranged downstream from the second collimator to beam-shape the arrangement radiation into beam-shaped radiation. The beam-shaping optical element may be a refractive optical element. The refractive optical element may comprise an array of refractive optical structures that may comprise semispherical lenses, spherical lenses, lens array(s), pyramid, wedge shaped optical structures or combinations thereof.

The beam-shaped radiation has a beam-shaped spatial light distribution. The beam-shaped radiation may have a narrower spatial light distribution than the arrangement radiation. Further, the beam-shaped radiation may have an elongated beam shape. In particular, the beam-shaped radiation may have an elongated beam shape, in particular an asymmetrical beam shape.

Especially, in embodiments a light transmissive material, such as the optical filter, has a light transmission in the range of 50-100 %, especially in the range of 70-100%, for light having a wavelength selected from the UV wavelength range. Herein, the UV wavelength range especially relates to light having a wavelength selected from the range 100-380 nm, more especially selected from the range 190-230 nm.

Especially, a light absorbing material has a light absorbance in the range of 50-100 %, especially in the range of 70-100%, for light having a wavelength selected from the UV wavelength range. As can be derived from the above, this may apply for a wavelength range of at least 100 nm, especially a wavelength range of at least 250 nm, such as a wavelength range of at least 300 nm (within the range of 380-780 nm).

The transmission T (or light permeability) can be determined by providing light at a specific wavelength with a first intensity I1 to the light transmissive material under perpendicular radiation and relating the intensity of the light I2 at that wavelength measured after transmission through the material, to the first intensity of the light provided at that specific wavelength to the material, thus T=I2/I1. Likewise, the reflectivity R can be determined by relating the intensity of the light I3 at that wavelength measured after reflection by the material, to the first intensity of the light I1 provided at that specific wavelength to the material. Thus R= I3/I1. The absorbance A may in embodiments be defined as A=1-(T+R) (see also E-208 and E-406 of the CRC Handbook of Chemistry and Physics, 20 69th edition, 1988-1989).

In specific embodiments, a material may be considered transmissive when the transmission of the radiation at a wavelength or in a wavelength range is larger than the reflectivity and absorbance (at that wavelength or in that wavelength range), thus when T>R and T>A, especially wherein TIR≧1.2 and T/A≥1.2. In specific embodiments, a material may be considered reflective when the reflectivity of the radiation at a wavelength or in a wavelength range is larger than the transmission and absorbance (at that wavelength or in that wavelength range), thus when R>T and R>A, especially wherein R/T≥1.2 and R/A≥1.2. In specific embodiments, a material may be considered absorbing when the absorbance of the radiation at a wavelength or in a wavelength range is larger than the transmission and reflectivity (at that wavelength or in that wavelength range), thus when A>T and A>R, especially wherein A/T≥1.2 and A/R≥1.2. Here, T, R, and A refer to percentages.

The radiation generating system according to the present invention may further comprise a white light source providing white light. The white light source may comprise solid state light source e.g. a LED light source such as for example a blue and/or UV phosphor converted LED, and/or RGB LEDs. The white light may have a colour temperature in a range from 2000 to 8000 K. Further, the white light may have a CRI of at least 80. In such an embodiment the controller may be arranged for controlling intensity (I) and/or colour temperature (CT) of the white light. The intensity I and the colour temperature CT may be controlled individually. The intensity I of the white light may be measured at the white light source, or at the light exit window of the radiation generating system.

The radiation generating system of the present invention may be arranged in a housing comprising a light exit window. In the context of the invention a light exit window is to be interpreted as any area, volume, or material which allow light to pass through it. The housing may be a hermetic housing. The housing may have any geometrical shape. The housing may be formed as a cuboid, where at least one face of the cuboid may act as a light exit window from where arrangement light can be emitted. The housing may further comprise an attachment surface arranged to be positioned on the surface to which the housing is to be attached, such as a ceiling, a floor, a wall, a table, or another suitable surface in a room.

The housing may further comprise an optical element arranged to direct arrangement light and/or white light towards the light exit window. The optical element may be a reflector. The reflector may comprise a specular reflective surface, which may be made of metal. The metal may be or may comprise aluminum, silver, or combinations thereof. The reflector provides excellent beam shaping properties, such that the intensity of and the exposure time to the disinfecting light is maximized.

The radiation generating system of the present invention may be a luminaire.

The radiation generating system may be configured to suspend from a ceiling of a room by a suspension arrangement or may be attached to the ceiling. Further, the radiation generating system may be arranged at any other surface within the room, such as on a wall, on the floor or on a surface of a piece of furniture.

A plurality of radiation generating systems may be arranged at different locations within the same room.

The system may especially be configured to generate system radiation. In an operational mode, the system radiation comprises at least part of the arrangement radiation, downstream of the optical arrangement. However, it is not excluded that being a final exit window of the system and the optical arrangement further optical elements may be available. The system radiation may comprise white light.

Especially, the system radiation may comprise at least radiation having the first wavelength λ₁. As indicated above, the light source may provide radiation having a ratio of the spectral powers at λ₁ and λ₂ defined as I_{1,1}/I_{1,2}. Downstream of the radiation unit, more especially downstream of the optical arrangement, this ratio may be indicated as I_{2,1}/I_{2,2}. This ratio may be larger than the ratio I_{1,1}/I_{1,2}, due to the optical arrangement, especially the optical filter and the second collimator. The system radiation, in one or more operational modes, generated by the system, may having a ratio of the spectral powers at λ₁ and λ₂ defined as I_{3,1}/I_{3,2}. The latter ratio may be the same as I_{2,1}/I_{2,2}, and will in general also be larger than I_{1,1}/I_{1,2}, as the radiation unit, especially the optical arrangement, may be used to change the (first) spectral power distribution of the light source to a more desirable spectral power distribution.

Hence, in embodiments the radiation generating system is especially configured to generate in an operational mode system radiation having radiation at the first wavelength λ₁, even more especially having a ratio of the spectral powers at λ₁ and λ2 defined as I3,1/I3,2, which is larger than a ratio of the spectral powers at λ₁ and λ2 defined as I_{1,1}/I_{1,2} of the light source radiation.

The radiation generating system may be part of or may be applied in e.g. office lighting systems, household application systems, shop lighting systems, home lighting systems, accent lighting systems, spot lighting systems, theater lighting systems, fiber-optics application systems, projection systems, self-lit display systems, pixelated display systems, segmented display systems, warning sign systems, medical lighting application systems, indicator sign systems, decorative lighting systems, portable systems, automotive applications, (outdoor) road lighting systems, urban lighting systems, green house lighting systems, horticulture lighting, digital projection, or LCD backlighting.

The present invention further relates to a method for treating at least part of a space or of an object, wherein the method comprises providing system radiation having the first wavelength λ₁ in the space or to the object using the radiation generating system as defined above. In particular, treating may refer to disinfection. In this way, viruses and/or bacteria may be killed using radiation that may be (relatively) safe for humans.

Considering the above, the present invention provides an improved radiation generating system that provides high safety level, allowing ongoing disinfection even when humans and/or animals are present in the area that is being disinfected. Moreover, the safety level of the radiation generating system allows positioning the system anywhere in the room, such as a wall or a table, which in turn provides an efficient disinfection.

### BRIEF DESCRIPTION OF THE DRAWINGS

Embodiments of the invention will now be described by way of example with reference to the accompanying drawings, of which:
Fig. 1a illustrates a radiation generating system according to the present invention;
Fig. 1b depicts a radiation generating system according to another embodiment of the present invention;
Figs. 2a-2e illustrate spatial light distribution after each element of the radiation generating system, wherein intensity of the radiation is plotted as a function of the angle.

All the figures are schematic, not necessarily to scale, and generally only show parts which are necessary in order to elucidate embodiments of the present invention, wherein other parts may be omitted or merely suggested.

### DETAILED DESCRIPTION

The present invention will now be described hereinafter with reference to the accompanying drawings, in which exemplifying embodiments of the present invention are shown. The present invention may, however, be embodied in many different forms and should not be construed as limited to the embodiments of the present invention set forth herein; rather, these embodiments of the present invention are provided by way of example so that this disclosure will convey the scope of the invention to those skilled in the art. In the drawings, identical or similar reference numerals denote the same or similar components having a same or similar function, unless specifically stated otherwise.

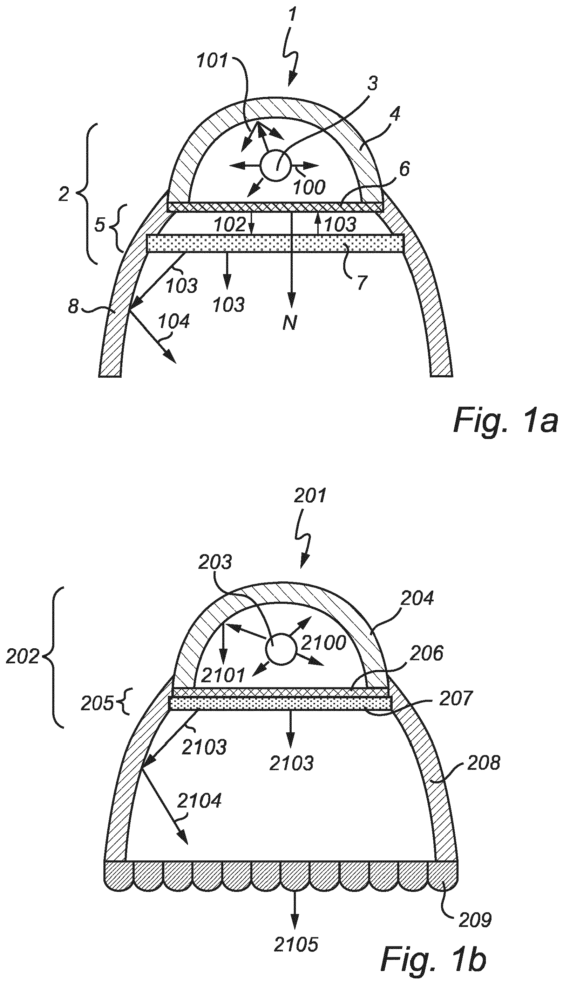

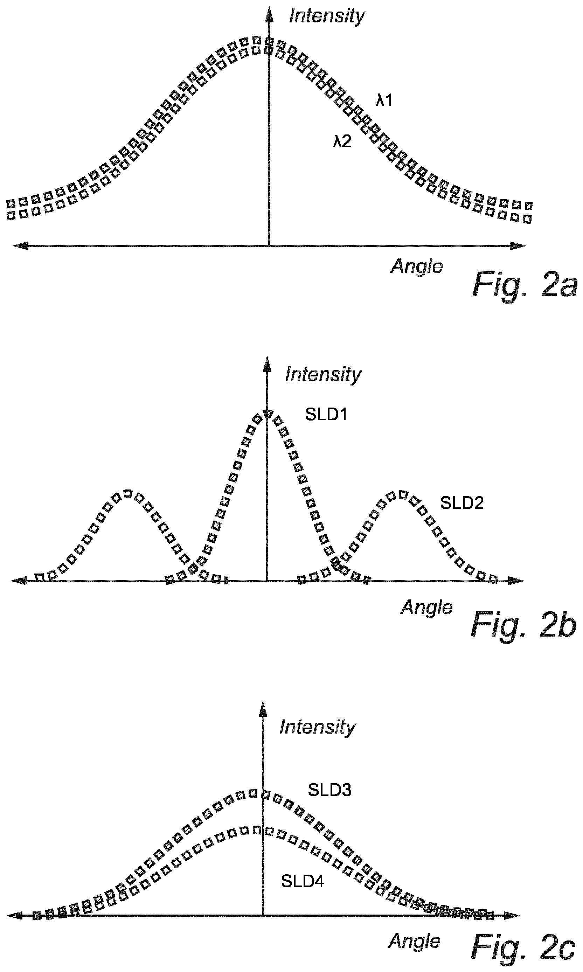

Fig. 1a illustrates a radiation generating system 1 comprising a radiation unit 2 comprising a light source 3, a first collimator 4, and an optical arrangement 5. The light source 3 is configured to generate light source radiation 100 having a first spectral power distribution having an intensity I_{1,1}, which may be referred to as a primary intensity, at a first wavelength λ₁ and an intensity I_{1,2}, referred to as a secondary intensity, at a second wavelength λ₂. The light source radiation 100 has a source spatial light distribution depicted in Fig. 2a. As mentioned above, the x-axis represents the incidence angle, while the y-axis represents intensity I_{1,1} at a first wavelength λ₁ and an intensity I_{1,2}, at a second wavelength λ₂. As may be seen in Fig. 2a, the intensities I_{1,1} and I_{1,2} of the light source radiation 100 are substantially equal.

The first collimator 4 is configured in a light receiving relationship with the light source 3, wherein the first collimator 4 is configured to collimate the light source radiation 100 into collimated light source radiation 101.

The first collimator 4 is configured upstream of the light source 3. The first collimator 4 comprises a reflective collimator, especially a light reflective collimator. The first collimator 4 may especially narrow the beam of radiation. The beam width may be expressed in the full width of the beam at half its maximum intensity (FWHM). The light source 3 may emit light source radiation 100 in substantially all directions. The first collimator 4 substantially redirects part of the light source radiation 100 into collimated light source radiation 101 directed to the optical arrangement 5. As may be seen in Fig. 1a, some light source radiation 100 may already be directed to the optical arrangement 5 and may not interact with the first collimator 4. Hence, the radiation that enters the optical arrangement 5 may comprise both light source radiation 100 and collimated light source radiation 101. Hence, the collimated light source radiation 101 is collimated relative to the light source radiation 100.

Further, the optical arrangement 5 of the radiation generating system 1 according to the present invention is configured downstream of the first collimator 4. The optical arrangement 5 is configured downstream of the first collimator 4 and the light source 3. As the first collimator 4 in embodiments may comprise a reflective collimator, the light source 3 is positioned in between the first collimator 4 and the optical arrangement 5. The optical arrangement 5 may receive the collimated light source radiation 101 from the first collimator 4, and optionally light source radiation 100 from the light source 3. The optical arrangement 5 comprises an optical filter 6 and a diffuser 7.

The optical filter 6 has a higher transmission for the first wavelength λ₁ than for the second wavelength λ₂, especially when irradiated under the predefined angle or range of angles. The optical filter 6 may comprise an interference filter. The interference filter may specifically transmit wavelengths in the range of 190-230 nm at the predefined angle. The interference filter may specifically absorb wavelengths in the range of 100-190 nm at the predefined angle. Additionally, or alternatively, the interference filter may specifically reflect wavelengths in the range of 100-190 nm at the predefined angle. Especially, the interference filter may have a lower transmission for a wavelength in the range of 100-190 nm. Additionally, or alternatively, the interference filter may specifically absorb wavelengths in the range of 230-280 nm at the predefined angle or have a lower transmission for a wavelength in such range.

The optical filter 6 is configured to filter at least part of the collimated light source radiation 101 into filtered radiation 102 comprising at least portion of the light source radiation 100 of the first wavelength λ₁ having a first spatial light distribution (SLD1) and at least portion of the light source radiation 100 of the second wavelength λ₂ having a second spatial light distribution (SLD2). As may be seen in Fig. 2b, there is only filtered radiation 102 at the first wavelength λ₁ at the angle θ=0°, since the optical filter 6 has reflected and/or absorbed the collimated light source radiation 101 at the second wavelength λ₂. However, at higher angles, the collimated light source radiation 101 at the second wavelength λ₂ is still present.

An optical axis O1 of the collimated light source radiation 101 may be aligned with a normal N to the optical filter 6. The optical axis O1 of the collimated light source radiation 101 may be defined as an imaginary axis that runs through a center of the light source 3 and through the second collimator 8. The normal N to the optical filter 6 may be defined as an axis perpendicular to a surface of the optical filter 6 configured for the incidence of the collimated radiation 101. Hence, the radiation generating system 1 is configured such that the optical axis O1 of the collimated light source radiation 101 and the normal (N) to the optical filter 6 have a first angle α1 equal to 0°.

The diffuser 7 is configured downstream of the optical filter 6. The diffuser 7 is configured to diffuse at least a part of the filtered radiation 102 into diffused radiation 103 comprising at least a portion of the light source radiation 100 of the first wavelength λ₁ having a third spatial light distribution (SLD3) and at least portion of the light source radiation 100 of the second wavelength λ₂ having a fourth spatial light distribution (SLD4) different from the second spatial light distribution (SLD2). In other words, the diffuser 7 redirects at least portion of the light source radiation 100 of the second wavelength λ₂, i.e. the harmful radiation, to another direction, such that this portion of radiation 103 may be collimated once more by the first collimator 4, and subsequently pass through the optical filter 6, wherein this radiation is filtered. SLD3 and SLD4 are shown in in Fig. 2c. As may be seen in Fig. 2c, the collimated light source radiation 101 at the second wavelength λ₂ has been redirected by the diffuser 7 from the higher angles back to a more narrow distribution, such that the collimated light source radiation 101 at the second wavelength λ₂ is filtered once more by the optical filter 6. At the same time, the filtered radiation 102 at the first wavelength λ₁ has been diffused to have a wider spatial light distribution.

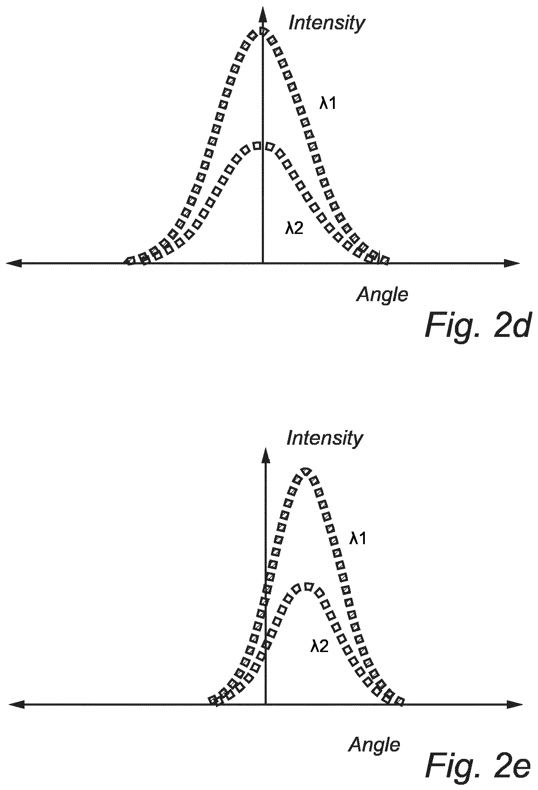

The optical arrangement 5 comprises a second collimator 8 being arranged downstream of the diffuser 7. The second collimator 8 may be a reflective collimator configured to collimate the diffused radiation 103 into arrangement radiation 104. The arrangement radiation 104 has an arrangement spatial light distribution depicted in Fig. 2d. As is evident from Fig. 2d, the second collimator 8 narrows the spatial light distribution of the arrangement radiation 104 compared to the diffused radiation 103. Further, it may be seen that the intensity of the radiation at the first wavelength λ₁ is greater than the intensity of the radiation at the first wavelength λ₂.

Fig. 1b illustrates a radiation generating system 201 comprising a radiation unit 202 comprising a light source 203, a first collimator 204, and an optical arrangement 205. The light source 203 is configured to generate light source radiation 2100 having a first spectral power distribution having an intensity I_{1,1}, which may be referred to as a primary intensity, at a first wavelength λ₁ and an intensity I_{1,2}, referred to as a secondary intensity, at a second wavelength λ₂. As may be seen in Fig. 2a, the intensities I_{1,1} and I_{1,2} of the light source radiation 2100 are substantially equal.

The first collimator 204 is configured in a light receiving relationship with the light source 3, wherein the first collimator 204 is configured to collimate the light source radiation 2100 into collimated light source radiation 2101.

The first collimator 204 is configured upstream of the light source 203. The first collimator 204 comprises a reflective collimator, especially a light reflective collimator. The first collimator 204 may especially narrow the beam of radiation. The beam width may be expressed in the full width of the beam at half its maximum intensity (FWHM). The light source 203 may emit light source radiation 2100 in substantially all directions. The first collimator 204 substantially redirects part of the light source radiation 2100 into collimated light source radiation 2101 directed to the optical arrangement 205. As may be seen in Fig. 1a, some light source radiation 2100 may already be directed to the optical arrangement 205 and may not interact with the first collimator 204. Hence, the radiation that enters the optical arrangement 205 may comprise both light source radiation 2100 and collimated light source radiation 2101. Hence, the collimated light source radiation 2101 is collimated relative to the light source radiation 2100.

Further, the optical arrangement 205 of the radiation generating system 201 according to the present invention is configured downstream of the first collimator 204. The optical arrangement 205 is configured downstream of the first collimator 204 and the light source 203. As the first collimator 204 in embodiments may comprise a reflective collimator, the light source 203 is positioned in between the first collimator 204 and the optical arrangement 205. The optical arrangement 205 may receive the collimated light source radiation 2101 from the first collimator 204, and optionally light source radiation 2100 from the light source 203. The optical arrangement 205 comprises an optical filter 206 and a diffuser 207.

The optical filter 206 has a higher transmission for the first wavelength λ₁ than for the second wavelength λ₂, especially when irradiated under the predefined angle or range of angles. The optical filter 6 may comprise an interference filter. The interference filter may specifically transmit wavelengths in the range of 190-230 nm at the predefined angle. The interference filter may specifically absorb wavelengths in the range of 100-190 nm at the predefined angle. Additionally, or alternatively, the interference filter may specifically reflect wavelengths in the range of 100-190 nm at the predefined angle. Especially, the interference filter may have a lower transmission for a wavelength in the range of 100-190 nm. Additionally, or alternatively, the interference filter may specifically absorb wavelengths in the range of 230-280 nm at the predefined angle or have a lower transmission for a wavelength in such range.

The optical filter 206 is configured to filter at least part of the collimated light source radiation 2101 into filtered radiation 2102 comprising at least portion of the light source radiation 2100 of the first wavelength λ₁ having a first spatial light distribution (SLD1) and at least portion of the light source radiation 2100 of the second wavelength λ₂ having a second spatial light distribution (SLD2). As may be seen in Fig. 2b, there is only filtered radiation 2102 at the first wavelength λ₁ at the angle θ=0°, since the optical filter 206 has reflected and/or absorbed the collimated light source radiation 2101 at the second wavelength λ₂. However, at higher angles, the collimated light source radiation 2101 at the second wavelength λ₂ is still present.

An optical axis O1 of the collimated light source radiation 2101 may be aligned with a normal N to the optical filter 206. The optical axis O1 of the collimated light source radiation 2101 may be defined as an imaginary axis that runs through a center of the light source 203 and through the second collimator 208. The normal N to the optical filter 206 may be defined as an axis perpendicular to a surface of the optical filter 206 configured for the incidence of the collimated radiation 2101. Hence, the radiation generating system 201 is configured such that the optical axis O1 of the collimated light source radiation 2101 and the normal (N) to the optical filter 206 have a first angle α1 equal to 0°.

The diffuser 207 is configured downstream of the optical filter 206. The diffuser 207 is configured to diffuse at least a part of the filtered radiation 2102 into diffused radiation 2103 comprising at least a portion of the light source radiation 2100 of the first wavelength λ₁ having a third spatial light distribution (SLD3) and at least portion of the light source radiation 2100 of the second wavelength λ₂ having a fourth spatial light distribution (SLD4) different from the second spatial light distribution (SLD2). In other words, the diffuser 207 redirects at least portion of the light source radiation 2100 of the second wavelength λ₂, i.e. the harmful radiation, to another direction, such that this portion of radiation 2103 may be collimated once more by the first collimator 204, and subsequently pass through the optical filter 206, wherein this radiation is filtered. SLD3 and SLD4 are shown in Fig. 2c. As may be seen in Fig. 2c, the collimated light source radiation 2101 at the second wavelength λ₂ has been redirected by the diffuser 207 from the higher angles back to a more narrow distribution, such that the collimated light source radiation 2101 at the second wavelength λ₂ is filtered once more by the optical filter 206. At the same time, the filtered radiation 2102 at the first wavelength λ₁ has been diffused to have a wider spatial light distribution.

The optical arrangement 205 comprises a second collimator 208 being arranged downstream of the diffuser 207. The second collimator 208 may be a reflective collimator configured to collimate the diffused radiation 2103 into arrangement radiation 2104. The arrangement radiation 2104 has an arrangement SLD depicted in Fig. 2d. As is evident from Fig. 2d, the second collimator 208 narrows the spatial light distribution of the arrangement radiation 2104 compared to the diffused radiation 2103. Further, it may be seen that the intensity of the radiation at the first wavelength λ₁ is greater than the intensity of the radiation at the first wavelength λ₂.

As may be seen in Fig. 1b, the diffuser 207 is in optical contact with the optical filter 106. In such an embodiment, the light loss due to reflection losses inherent to difference in refractive index with air are eliminated. Alternatively, or additionally, adhesive may be applied between the diffuser and the optical filter.

The radiation generating system 201 shown in Fig. 1b comprises a beam-shaping optical element 209 being arranged downstream from the second collimator 204 to beam-shape the arrangement radiation 2104 into beam-shaped radiation 2105. The beam-shaping optical element 209 is a refractive optical element comprising an array of refractive optical structures that may comprise semispherical lenses.

The beam-shaped radiation 2105 may have a narrower spatial light distribution than the arrangement radiation 2104. The beam-shaped radiation 2105 has a beam-shaped spatial light distribution shown in Fig. 2e.

Although the present invention has been described with reference to various embodiments, those skilled in the art will recognize that changes may be made without departing from the scope of the invention. as long as they fall within the scope of the claims. It is intended that the detailed description be regarded as illustrative and that the appended claims including all the equivalents are intended to define the scope of the invention. While the present invention has been illustrated in the appended drawings and the foregoing description, such illustration is to be considered illustrative or exemplifying and not restrictive; the present invention is not limited to the disclosed embodiments. Other variations to the disclosed embodiments can be understood and effected by those skilled in the art in practicing the claimed invention, from a study of the drawings, the disclosure, and the appended claims. **In** the appended claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A radiation generating system (1) comprising a radiation unit (2), wherein said radiation unit (2) comprises a light source (3), a first collimator (4), and an optical arrangement (5), wherein:
- said light source (3) is configured to generate light source radiation (100) having a first spectral power distribution having an intensity I_{1,1} at a first wavelength λ₁ and an intensity I_{1,2} at a second wavelength λ₁
- said first collimator (4) is configured in a light receiving relationship with said light source (3), wherein said first collimator (4) is configured to collimate said light source radiation (100) into collimated light source radiation (101) having an initial spatial light distribution (ISLD);
- said optical arrangement (5) is arranged downstream of said first collimator (4) and comprises an optical filter (6) and a diffuser (7), wherein:
- said optical filter (6) has a higher transmission for said first wavelength λ₁ than for said second wavelength λ₂ when irradiated under a predefined angle θ;
- said first wavelength λ₁ is selected from the range of 190-230 nm and said second wavelength λ₂ is selected from the range of 100-190 nm and/or 230-300 nm;
- said optical filter (6) is configured to filter at least part of said collimated light source radiation (101) into filtered radiation (102) comprising at least portion of said light source radiation (100) of said first wavelength λ₁ having a first spatial light distribution (SLD1) and at least portion of said light source radiation (100) of said second wavelength λ₂ having a second spatial light distribution (SLD2) different from said first spatial light distribution (SLD1);
- said diffuser (7) is configured downstream of said optical filter (6);
- said diffuser (7) is configured to diffuse at least part of said filtered radiation (102) into diffused radiation (103) comprising at least a portion of said light source radiation (100) of said first wavelength λ₁ having a third spatial light distribution (SLD3) and at least portion of said light source radiation (100) of said second wavelength λ₂ having a fourth spatial light distribution (SLD4) different from said second spatial light distribution (SLD2),
**characterised in that**
said radiation generating system (1) further comprises a second collimator (8) being arranged downstream of said diffuser (7); wherein said second collimator (8) is a reflective collimator configured to collimate said diffused radiation (103) into arrangement radiation (104) having a narrower spatial light distribution than said diffused radiation.

2. The radiation generating system (201) according to claim 1, wherein a beam-shaping optical element (209) is arranged downstream said second collimator (208) to beam-shape said arrangement radiation (2104) into beam-shaped radiation (2105).

3. The radiation generating system (201) according to claim 2, wherein said beam-shaping optical element (209) is a refractive optical element.

4. The radiation generating system (201) according to claim 3, wherein said refractive optical element comprises an array of refractive optical structures.

5. The radiation generating system (201) according to any one of claims 2-4, wherein said beam-shaped radiation (2105) has a narrower spatial light distribution than said arrangement radiation (104).

6. The radiation generating system (201) according to any one of claims 2-5, wherein said beam-shaped radiation (2105) has an elongated beam shape.

7. The radiation generating system (1) according to any one of the preceding claims, wherein 2≤ I_{1,1}/I_{1,2}≤100.

8. The radiation generating system (1) according to any one of the preceding claims, wherein the reflectivity (R) of the diffuser is in the range from 15% to 35% for said light source radiation.

9. The radiation generating system (201) according to any one of the preceding claims, wherein said diffuser (207) is in optical contact with said optical filter (206).

10. The radiation generating system (1) according to any one of the preceding claims, wherein (i) the dominant peak wavelength of said first wavelength λ₁ is 207 nm and/or 222 nm, and/or (ii) the dominant peak wavelength of said second wavelength λ₂ is in a wavelength range from 285 nm to 295 nm and/or from 255 to 260 nm.

11. The radiation generating system (1) according to any one of the preceding claims, wherein said first collimator (4) is a light mixing chamber or a reflector.

12. The radiation generating system (1) according to any one of the preceding claims, wherein said optical filter (6) has a transmittance characteristic with a high transmission for said first wavelength λ₁ for the predefined angle θ with θ in an angle range of -30 to +30° with respect to the normal to said filter, and wherein said optical filter (6) has substantially no transmittance for said second wavelength λ₂ in said angle range of -30 to +30° with respect to the normal to the filter.

13. The radiation generating system (1) according to any one of the preceding claims, wherein said light source (3) comprises an excimer discharge based light source.

14. A method for treating at least part of a space or of an object, wherein said method comprises providing system radiation having said first wavelength λ₁ in a space or to an object using said radiation generating system (1) as defined in any one of the preceding claims.

## Patentansprüche

1. Strahlungserzeugungssystem (1), umfassend eine Strahlungseinheit (2), wobei die Strahlungseinheit (2) eine Lichtquelle (3), einen ersten Kollimator (4) und eine optische Anordnung (5) umfasst, wobei:
- die Lichtquelle (3) konfiguriert ist, um Lichtquellenstrahlung (100) zu erzeugen, die eine erste spektrale Leistungsverteilung aufweist, die eine Intensität I_{1,1} bei einer ersten Wellenlänge λ₁ und eine Intensität I_{1,2} bei einer zweiten Wellenlänge λ₂ aufweist;
- der erste Kollimator (4) in einer lichtempfangenden Beziehung mit der Lichtquelle (3) konfiguriert ist, wobei der erste Kollimator (4) konfiguriert ist, um die Lichtquellenstrahlung (100) in kollimierte Lichtquellenstrahlung (101) zu kollimieren, die eine anfängliche räumliche Lichtverteilung (ISLD) aufweist;
- die optische Anordnung (5) dem ersten Kollimator (4) nachgeschaltet angeordnet ist und einen optischen Filter (6) und einen Diffusor (7) umfasst, wobei:
- der optische Filter (6) eine höhere Transmission für die erste Wellenlänge λ₁ als für die zweite Wellenlänge λ₂ aufweist, wenn unter einem vordefinierten Winkel θ bestrahlt;
- die erste Wellenlänge λ₁ aus dem Bereich von 190-230 nm ausgewählt ist und die zweite Wellenlänge λ₂ aus dem Bereich von 100-190 nm und/oder 230-300 nm ausgewählt ist;
- der optische Filter (6) konfiguriert ist, um mindestens einen Teil der kollimierten Lichtquellenstrahlung (101) in gefilterte Strahlung (102) zu filtern, umfassend mindestens einen Anteil der Lichtquellenstrahlung (100) der ersten Wellenlänge λ₁, die eine erste räumliche Lichtverteilung (SLD1) aufweist, und mindestens einen Anteil der Lichtquellenstrahlung (100) der zweiten Wellenlänge λ₂, die eine zweite räumliche Lichtverteilung (SLD2) aufweist, die sich von der ersten räumlichen Lichtverteilung (SLD1) unterscheidet;
- der Diffusor (7) nachgeschaltet des optischen Filters (6) konfiguriert ist;
- der Diffusor (7) konfiguriert ist, um einen Teil der gefilterten Strahlung (102) in diffundierte Strahlung (103) zu diffundieren, umfassend mindestens einen Anteil der Strahlung der Lichtquelle (100) der ersten Wellenlänge λ₁, die eine dritte räumliche Lichtverteilung (SLD3) aufweist, und mindestens einen Anteil der Lichtquellenstrahlung (100) der zweiten Wellenlänge λ₂, die eine vierte räumliche Lichtverteilung (SLD4), aufweist die sich von der zweiten räumlichen Lichtverteilung (SLD2) unterscheidet,
**dadurch gekennzeichnet, dass** das Strahlungserzeugungssystem (1) ferner einen zweiten Kollimator (8) umfasst, der nachgeschaltet des Diffusors (7) angeordnet ist; wobei der zweite Kollimator (8) ein reflektierender Kollimator ist, der konfiguriert ist, um die diffundierte Strahlung (103) in eine Anordnungsstrahlung (104) zu kollimieren, die eine engere räumliche Lichtverteilung als die diffundierte Strahlung aufweist.

2. Strahlungserzeugungssystem (201) nach Anspruch 1, wobei ein strahlformendes optisches Element (209) nachgeschaltet des zweiten Kollimators (208) angeordnet ist, um die Anordnungsstrahlung (2104) in strahlgeformte Strahlung (2105) als Strahl zu formen.

3. Strahlungserzeugungssystem (201) nach Anspruch 2, wobei das strahlformende optische Element (209) ein refraktives optisches Element ist.

4. Strahlungserzeugungssystem (201) nach Anspruch 3, wobei das refraktive optische Element ein Array von refraktiven optischen Strukturen umfasst.

5. Strahlungserzeugungssystem (201) nach einem der Ansprüche 2 bis 4, wobei die strahlförmige Strahlung (2105) eine engere räumliche Lichtverteilung als die Anordnungsstrahlung (104) aufweist.

6. Strahlungserzeugungssystem (201) nach einem der Ansprüche 2 bis 5, wobei die strahlförmige Strahlung (2105) eine längliche Strahlform aufweist.

7. Strahlungserzeugungssystem (1) nach einem der vorstehenden Ansprüche, wobei 2≤I_{1,1}/I_{1,2}≤100.

8. Strahlungserzeugungssystem (1) nach einem der vorstehenden Ansprüche, wobei die Reflektivität (R) des Diffusors für die Lichtquellenstrahlung in dem Bereich von 15 % bis 35 % liegt.

9. Strahlungserzeugungssystem (201) nach einem der vorstehenden Ansprüche, wobei der Diffusor (207) in optischem Kontakt mit dem optischen Filter (206) steht.

10. Strahlungserzeugungssystem (1) nach einem der vorstehenden Ansprüche, wobei (i) die farbtongleiche Spitzenwellenlänge der ersten Wellenlänge λ₁ 207 nm und/oder 222 nm beträgt und/oder (ii) die farbtongleiche Spitzenwellenlänge der zweiten Wellenlänge λ₂ in einem Wellenlängenbereich von 285 nm bis 295 nm und/oder von 255 bis 260 nm liegt.

11. Strahlungserzeugungssystem (1) nach einem der vorstehenden Ansprüche, wobei der erste Kollimator (4) eine Lichtmischkammer oder ein Reflektor ist.

12. Strahlungserzeugungssystem (1) nach einem der vorstehenden Ansprüche, wobei der optische Filter (6) eine Transmissionscharakteristik mit einer hohen Transmission für die erste Wellenlänge λ₁ für den vordefinierten Winkel θ mit θ in einem Winkelbereich von -30 bis +30° in Bezug auf die Normale zu dem Filter aufweist, und wobei der optische Filter (6) im Wesentlichen keine Transmission für die zweite Wellenlänge λ₂ in dem Winkelbereich von -30 bis +30° in Bezug auf die Normale zu dem Filter aufweist.

13. Strahlungserzeugungssystem (1) nach einem der vorstehenden Ansprüche, wobei die Lichtquelle (3) eine auf einer Excimer-Entladung basierende Lichtquelle umfasst.

14. Verfahren zum Behandeln von mindestens einem Teil eines Raumes oder eines Objekts, wobei das Verfahren das Bereitstellen von Systemstrahlung, die die erste Wellenlänge λ₁ aufweist, in einem Raum oder an einem Objekt unter Verwendung des Strahlungserzeugungssystems (1) nach einem der vorstehenden Ansprüche umfasst.

## Revendications

1. Système de génération de rayonnement (1) comprenant une unité de rayonnement (2), dans lequel ladite unité de rayonnement (2) comprend une source de lumière (3), un premier collimateur (4) et un agencement optique (5), dans lequel :
- ladite source de lumière (3) est configurée pour générer un rayonnement de source de lumière (100) ayant une première distribution de puissance spectrale ayant une intensité I_{1,1} à une première longueur d'onde λ₁ et une intensité I_{1,2} à une seconde longueur d'onde λ₂ ;
- ledit premier collimateur (4) est configuré dans une relation de réception de lumière avec ladite source de lumière (3), dans lequel ledit premier collimateur (4) est configuré pour collimater ledit rayonnement de source de lumière (100) en un rayonnement de source de lumière collimatée (101) ayant une distribution spatiale de lumière initiale (ISLD) ;
- ledit agencement optique (5) est agencé en aval dudit premier collimateur (4) et comprend un filtre optique (6) et un diffuseur (7), dans lequel :
- ledit filtre optique (6) a une transmission pour ladite première longueur d'onde λ₁ plus élevée que pour ladite seconde longueur d'onde λ₂ lorsqu'il est irradié selon un angle prédéfini θ ;
- ladite première longueur d'onde λ₁ est sélectionnée dans la plage de 190 à 230 nm et ladite seconde longueur d'onde λ₂ est sélectionnée dans la plage de 100 à 190 nm et/ou 230 à 300 nm ;
- ledit filtre optique (6) est configuré pour filtrer au moins une partie dudit rayonnement de source de lumière collimatée (101) en un rayonnement filtré (102) comprenant au moins une portion dudit rayonnement de source de lumière (100) de ladite première longueur d'onde λ₁ ayant une première distribution spatiale de lumière (SLD1) et au moins une portion dudit rayonnement de source de lumière (100) de ladite seconde longueur d'onde λ₂ ayant une deuxième distribution spatiale de lumière (SLD2) différente de ladite première distribution spatiale de lumière (SLD1) ;
- ledit diffuseur (7) est configuré en aval dudit filtre optique (6) ;
- ledit diffuseur (7) est configuré pour diffuser au moins une partie dudit rayonnement filtré (102) en un rayonnement diffusé (103) comprenant au moins une portion dudit rayonnement de source de lumière (100) de ladite première longueur d'onde λ₁ ayant une troisième distribution spatiale de lumière (SLD3) et au moins une portion dudit rayonnement de source de lumière (100) de ladite seconde longueur d'onde λ₂ ayant une quatrième distribution spatiale de lumière (SLD4) différente de ladite deuxième distribution spatiale de lumière (SLD2),
**caractérisé en ce que** ledit système de génération de rayonnement (1) comprend en outre un second collimateur (8) étant agencé en aval dudit diffuseur (7) ; dans lequel ledit second collimateur (8) est un collimateur réfléchissant configuré pour collimater ledit rayonnement diffusé (103) en un rayonnement d'agencement (104) ayant une distribution spatiale de lumière plus étroite que ledit rayonnement diffusé.

2. Système de génération de rayonnement (201) selon la revendication 1, dans lequel un élément optique de mise en forme de faisceau (209) est agencé en aval dudit second collimateur (208) pour mettre en forme le faisceau dudit rayonnement d'agencement (2104) en un rayonnement à faisceau mis en forme (2105).

3. Système de génération de rayonnement (201) selon la revendication 2, dans lequel ledit élément optique de mise en forme de faisceau (209) est un élément optique réfringent.

4. Système de génération de rayonnement (201) selon la revendication 3, dans lequel ledit élément optique réfringent comprend une matrice de structures optiques réfringentes.

5. Système de génération de rayonnement (201) selon l'une quelconque des revendications 2 à 4, dans lequel ledit rayonnement à faisceau mis en forme (2105) a une distribution spatiale de lumière plus étroite que ledit rayonnement d'agencement (104).

6. Système de génération de rayonnement (201) selon l'une quelconque des revendications 2 à 5, dans lequel ledit rayonnement à faisceau mis en forme (2105) a une forme de faisceau allongée.

7. Système de génération de rayonnement (1) selon l'une quelconque des revendications précédentes, dans lequel 2≤ I_{1,1}/I_{1,2}≤100.

8. Système de génération de rayonnement (1) selon l'une quelconque des revendications précédentes, dans lequel la réflectivité (R) du diffuseur est dans la plage allant de 15 % à 35 % pour ledit rayonnement de source de lumière.

9. Système de génération de rayonnement (201) selon l'une quelconque des revendications précédentes, dans lequel ledit diffuseur (207) est en contact optique avec ledit filtre optique (206).

10. Système de génération de rayonnement (1) selon l'une quelconque des revendications précédentes, dans lequel (i) la longueur d'onde de pic dominant de ladite première longueur d'onde λ₁ est de 207 nm et/ou 222 nm, et/ou (ii) la longueur d'onde de pic dominant de ladite seconde longueur d'onde λ₂ est dans une plage de longueur d'onde allant de 285 nm à 295 nm et/ou de 255 à 260 nm.

11. Système de génération de rayonnement (1) selon l'une quelconque des revendications précédentes, dans lequel ledit premier collimateur (4) est une chambre de mélange de lumière ou un réflecteur.

12. Système de génération de rayonnement (1) selon l'une quelconque des revendications précédentes, dans lequel ledit filtre optique (6) a une caractéristique de transmittance avec une transmission élevée pour ladite première longueur d'onde λ₁ pour l'angle prédéfini θ avec θ dans une plage angulaire de -30 à +30° par rapport à la normale audit filtre, et dans lequel ledit filtre optique (6) n'a sensiblement aucune transmittance pour ladite seconde longueur d'onde λ₂ dans ladite plage angulaire de -30 à +30° par rapport à la normale au filtre.

13. Système de génération de rayonnement (1) selon l'une quelconque des revendications précédentes, dans lequel ladite source de lumière (3) comprend source de lumière à base de décharge d'excimère.

14. Procédé permettant de traiter au moins une partie d'un espace ou d'un objet, dans lequel ledit procédé comprend la fourniture d'un rayonnement de système ayant ladite première longueur d'onde λ₁ dans un espace ou sur un objet à l'aide dudit système de génération de rayonnement (1) selon l'une quelconque des revendications précédentes.
